# EUROPÄISCHE PATENTANMELDUNG

(11) **EP 1 104 812 A1**
(43) Veröffentlichungstag der Anmeldung: **06.06.2001**
(21) Anmeldenummer: 00123955.7
(22) Anmeldetag: 03.11.2000
(51) Int. Cl.: C12N 15/61, C12N 9/90, C12P 13/04, C12P 13/08

(54) **Für das gpm-Gen kodierende Nukleotidsequenzen**

(30) Priorität: 02.12.1999 DE 19958160
(71) Anmelder: Degussa AG, 40474 Düsseldorf (DE)
(72) Erfinder: Möckel, Bettina, Dr., 40597 Düsseldorf (DE); Pfefferle, Walter, Dr., 33790 Halle (Westf.) (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c),
und Verfahren zur fermentativen Herstellung von L-Aminosäuren unter Verstärkung des gpm-Gens.

SEQUENZPROTOKOLL

<110> Degussa-Hüls AG

<120> Neue für das gpm-Gen kodierende Nukleotidsequenzen

<130> 990168 BT

<140>
<141>

<160> 2

<170> PatentIn Ver. 2.1

<210> 1
<211> 1020
<212> DNA
<213> Corynebacterium glutamicum

<220>
<221> CDS
<222> (181)..(924)

<400> 1
acgcgcatca gaatgggtga agacgccgtc gaacacgcca gaacattctc ctgggcggcc 60

accgccgcac agctatcgtc gctgtacaac gacgctattg ccaacgaaaa tgtcgacggt 120

gaaacgcatc acggctaagt aaacgcgcgt cgtggaacat aaagtggcaa actagtacct 180

atg act aac gga aaa ttg att ctt ctt cgt cac ggt cag agc gaa tgg 228
Met Thr Asn Gly Lys Leu Ile Leu Leu Arg His Gly Gln Ser Glu Trp
  1               5                  10                  15

aac gca tcc aac cag ttc act gga tgg gtc gac gtc aat ctg acc gaa 276
Asn Ala Ser Asn Gln Phe Thr Gly Trp Val Asp Val Asn Leu Thr Glu
             20                  25                  30

cag ggt gag gct gag gcc aag cgc gga ggc gaa ctc ctc gtc gag gca 324
Gln Gly Glu Ala Glu Ala Lys Arg Gly Gly Glu Leu Leu Val Glu Ala
         35                  40                  45

ggc gtc ctc cca ggc gtt gta tac acc tcc ttg ctg cgt cgc gcg atc 372
Gly Val Leu Pro Gly Val Val Tyr Thr Ser Leu Leu Arg Arg Ala Ile
     50                  55                  60

cgc act gca aac atc gca ctg aac gct gca gac cgc cac tgg atc cca 420
Arg Thr Ala Asn Ile Ala Leu Asn Ala Ala Asp Arg His Trp Ile Pro
 65                  70                  75                  80

gtg atc cgc gac tgg cgc ctc aac gag cgt cac tac ggc gca ctg cag 468
Val Ile Arg Asp Trp Arg Leu Asn Glu Arg His Tyr Gly Ala Leu Gln
                 85                  90                  95

ggc ctt gac aag gct gca acc aag gaa aaa tac ggc gac gac cag ttc 516
Gly Leu Asp Lys Ala Ala Thr Lys Glu Lys Tyr Gly Asp Asp Gln Phe
            100                 105                 110

atg gaa tgg cgc cgc tcc tac gac acc cca cca cca gag ctc gcg gat 564
Met Glu Trp Arg Arg Ser Tyr Asp Thr Pro Pro Pro Glu Leu Ala Asp
        115                 120                 125

gac gca gag tac tcc cag gca aat gac cct cgt tac gcg gac ctc gac 612
Asp Ala Glu Tyr Ser Gln Ala Asn Asp Pro Arg Tyr Ala Asp Leu Asp
    130                 135                 140

gta gtt cca cgc acc gaa tgc ctc aag gac gtt gtg gtt cgt ttt gtt 660
Val Val Pro Arg Thr Glu Cys Leu Lys Asp Val Val Val Arg Phe Val
145                 150                 155                 160

cct tac ttc gag gaa gaa atc ctg cca cgc gca aag aag ggc gaa acc 708
Pro Tyr Phe Glu Glu Glu Ile Leu Pro Arg Ala Lys Lys Gly Glu Thr
                165                 170                 175

gtc ctc atc gca gca cac ggc aac tcc ctg cgt gcg ctg gtt aag cac 756
Val Leu Ile Ala Ala His Gly Asn Ser Leu Arg Ala Leu Val Lys His
            180                 185                 190

ctt gac ggc atc tcc gat gct gat atc gca gag ctc aac atc cca acc 804
Leu Asp Gly Ile Ser Asp Ala Asp Ile Ala Glu Leu Asn Ile Pro Thr
        195                 200                 205

ggc atc cca ctg gtc tac gaa atc gcc gaa gac ggt tcc gta gta aac 852
Gly Ile Pro Leu Val Tyr Glu Ile Ala Glu Asp Gly Ser Val Val Asn
    210                 215                 220

cca ggc ggc acc tac ctc gat cct gag gca gca gca gcc ggc gca gca 900
Pro Gly Gly Thr Tyr Leu Asp Pro Glu Ala Ala Ala Ala Gly Ala Ala
225                 230                 235                 240

gca gta gca aac cag ggt aat aag tagctatttg taggtgagca ctcttcttgc 954
Ala Val Ala Asn Gln Gly Asn Lys
                245

tttcgtattg ggcgtggtcc tcatgggcct cgccctacct gcgtatacga aaattaaaga 1014

tcggat 1020

<210> 2
<211> 248
<212> PRT
<213> Corynebacterium glutamicum

<400> 2
Met Thr Asn Gly Lys Leu Ile Leu Leu Arg His Gly Gln Ser Glu Trp
1               5                   10                  15

Asn Ala Ser Asn Gln Phe Thr Gly Trp Val Asp Val Asn Leu Thr Glu
            20                  25                  30

Gln Gly Glu Ala Glu Ala Lys Arg Gly Gly Glu Leu Leu Val Glu Ala
        35                  40                  45

Gly Val Leu Pro Gly Val Val Tyr Thr Ser Leu Leu Arg Arg Ala Ile
    50                  55                  60

Arg Thr Ala Asn Ile Ala Leu Asn Ala Ala Asp Arg His Trp Ile Pro
65                  70                  75                  80

Val Ile Arg Asp Trp Arg Leu Asn Glu Arg His Tyr Gly Ala Leu Gln
                85                  90                  95

Gly Leu Asp Lys Ala Ala Thr Lys Glu Lys Tyr Gly Asp Asp Gln Phe
            100                 105                 110

Met Glu Trp Arg Arg Ser Tyr Asp Thr Pro Pro Pro Glu Leu Ala Asp
        115                 120                 125

Asp Ala Glu Tyr Ser Gln Ala Asn Asp Pro Arg Tyr Ala Asp Leu Asp
    130                 135                 140

Val Val Pro Arg Thr Glu Cys Leu Lys Asp Val Val Val Arg Phe Val
145                 150                 155                 160

Pro Tyr Phe Glu Glu Glu Ile Leu Pro Arg Ala Lys Lys Gly Glu Thr
                165                 170                 175

Val Leu Ile Ala Ala His Gly Asn Ser Leu Arg Ala Leu Val Lys His
            180                 185                 190

Leu Asp Gly Ile Ser Asp Ala Asp Ile Ala Glu Leu Asn Ile Pro Thr
        195                 200                 205

Gly Ile Pro Leu Val Tyr Glu Ile Ala Glu Asp Gly Ser Val Val Asn
    210                 215                 220

Pro Gly Gly Thr Tyr Leu Asp Pro Glu Ala Ala Ala Ala Gly Ala Ala
225                 230                 235                 240

Ala Val Ala Asn Gln Gly Asn Lys
                245

## Beschreibung

Gegenstand der Erfindung sind für das gpm-Gen kodierende Nukleotidsequenzen und Verfahren zur fermentativen Herstellung von Aminosäuren, insbesondere L-Lysin unter Verwendung von coryneformen Bakterien, in denen das gpm-Gen verstärkt wird.

### Stand der Technik

Aminosäuren, insbesondere L-Lysin, finden in der Humanmedizin und in der pharmazeutischen Industrie, insbesondere aber in der Tierernährung Anwendung.

Es ist bekannt, daß Aminosäuren durch Fermentation von Stämmen coryneformer Bakterien, insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Lysin-Analogon S-(2-Aminoethyl)-Cystein oder auxotroph für regulatorisch bedeutsame Metabolite sind und L-Lysin produzieren.

Seit einigen Jahren werden ebenfalls Methoden der rekombinanten DNA-Technik zur Stammverbesserung Aminosäure produzierender Stämme von Corynebacterium eingesetzt, indem man einzelne Aminosäure-Biosynthesegene amplifiziert und die Auswirkung auf die Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von Aminosäuren insbesondere L-Lysin bereitzustellen.

### Beschreibung der Erfindung

Aminosäuren insbesondere L-Lysin finden in der Humanmedizin, in der pharmazeutischen Industrie und insbesondere in der Tierernährung Anwendung. Es besteht daher ein allgemeines Interesse daran, neue verbesserte Verfahren zur Herstellung von Aminosäuren insbesondere L-Lysin bereitzustellen.

Wenn im folgenden L-Lysin oder Lysin erwähnt werden, sind damit nicht nur die Base sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ ID No.1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

### Weitere Gegenstände sind

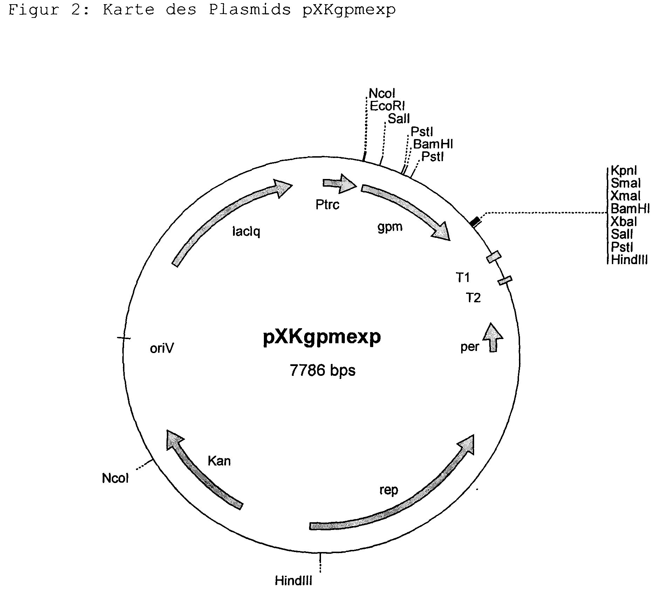

ein Polynukleotid gemäß Anspruch 4, enthaltend die Nukleotidsequenz wie in SEQ ID No. 1 dargestellt,
ein Polynukleotid gemäß Anspruch 6, das für ein Polypeptid kodiert, das die Aminosäuresequenz, wie in SEQ ID No. 2 dargestellt, enthält
ein Vektor, enthaltend das Polypeptid gemäß Anspruch 1, insbesondere Pendelvektor oder Plasmidvektor pXKgpmexp, der in Figur 2 dargestellt ist.
und als Wirtszelle dienende coryneforme Bakterien, die den Vektor enthalten oder in denen das gpm-Gen verstärkt wird.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthält, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind geeignet als Hybridisierungs-Sonden für RNA, cDNA und DNA, um cDNA in voller Länge zu isolieren, die für Phosphoglycerate Mutase kodieren und solche cDNA oder Gene zu isolieren, die eine hohe Ähnlichkeit der Sequenz mit der des Phosphoglycerate Mutase-Gens aufweisen.

Polynukleotidsequenzen gemäß der Erfindung sind weiterhin geeignet als Primer zur Herstellung von DNA von Genen, die für Phosphoglycerate Mutase kodieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Basen. Geeignet sind ebenfalls Oligonukleotide mit einer Länge von mindestens 40 oder 50 Basenpaaren.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität der Phosphoglycerate Mutase und auch solche ein, die zu wenigstens 70 % identisch sind mit dem Polypeptid gemäß SEQ ID No. 2, bevorzugt zu wenigstens 80% und besonders die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von Aminosäuren, insbesondere L-Lysin, unter Verwendung von coryneformen Bakterien, die insbesondere bereits eine Aminosäure produzieren, und in denen die für das gpm-Gen kodierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren, insbesondere L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die zum Beispiel bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715.

Den Erfindern gelang es, das neue, für das Enzym Phosphoglycerate Mutase (EC 5.4.2.1) kodierende gpm-Gen von C. glutamicum zu isolieren.

Zur Isolierung des gpm-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Viera et al., 1982, Gene, 19:259-268) verwendet werden. Als Wirte eignen sich besonders solche E. coliStämme, die restriktions- und rekombinationsdefekt sind. Ein Beispiel hierfür ist der Stamm DH5amcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige für die Sequenzierung geeignete Vektoren subkloniert und anschließend sequenziert werden, so wie es z. B. bei Sanger et al. (Proceedings of the National Academy of Sciences of the United States of America, 74:5463-5467, 1977) beschrieben ist.

Auf diese Weise wurde die neue für das Gen gpm kodierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des gpm-Genproduktes dargestellt.

Kodierende DNA-Sequenzen, die sich aus SEQ ID No. 1 durch die Degeneriertheit des genetischen Codes ergeben, sind ebenfalls Bestandteil der Erfindung. In gleicher Weise sind DNA-Sequenzen, die mit SEQ ID No. 1 oder Teilen von SEQ ID No. 1 hybridisieren Bestandteil der Erfindung. In der Fachwelt sind weiterhin konservative Aminosäureaustausche wie z. B. Austausch von Glycin gegen Alanin oder von Asparaginsäure gegen Glutaminsäure in Proteinen als ,,Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO. 2 ergeben, sind ebenfalls Bestandteil der Erfindung.

In gleicher Weise sind DNA-Sequenzen, die mit SEQ ID No. 1 oder Teilen von SEQ ID No. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben. Derartige Oligonukleotide haben typischerweise eine Länge von mindestens 15 Basenpaaren.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des gpm-Gens in verbesserter Weise Aminosäuren insbesondere L-Lysin produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße gpm-Gen mit Hilfe von Plasmiden überexprimiert.

Als Plasmide eignen sich solche, die in coryneformen Bakterien repliziert werden. Zahlreiche bekannte Plasmidvektoren wie z. B. pZl (Menkel et al., Applied and Environmental Microbiology (1989) 64: 549-554), pEKExl (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGAl. Andere Plasmidvektoren wie z. B. solche, die auf pCG4 (US-A 4,489,160) oder pNG2 (Serwold-Davis et al., FEMS Microbiology Letters 66, 119-124 (1990)) oder pAGl (US-A 5,158,891) beruhen, können in gleicher Weise verwendet werden.

Ein Beispiel für ein Plasmid, mit Hilfe dessen das gpm-Gen überexprimiert werden kann, ist der E.coli-C.glutamicum Shuttle-Expressionsvektor pXKgpmexp. Der Vektor enthält die Replikationsregion rep des Plasmids pGA1 einschließlich des Replikationseffectors per (US-A- 5,175,108; Nesvera et al., Journal of Bacteriology 179, 1525-1532 (1997)), das Kanamycin-Resistenzgen aph(3')-IIa aus Escherichia coli, den Replikationsursprung, den trc-Promotor, die Terminationsregionen T1 und T2, das lacI^{q}-Gen (Repressor des lac-Operons von E.coli) und eine Mehrfachklonierschnittstelle (multiple cloning site, mcs) (Norrander, J.M. et al. Gene 26, 101-106 (1983)) des Plasmids pTRC99A (Amann et al. (1988), Gene 69: 301-315).

Der Shuttle-Expressionsvektor pXKgpmexp ist in Figur 2 dargestellt.

Zusätzlich kann es für die Produktion von Aminosäuren, insbesondere L-Lysin, vorteilhaft sein, neben dem gpm-Gen eines oder mehrere Enzyme des jeweiligen Biosyntheseweges, der Glykolyse, der Anaplerotik, des Zitronensäure-Zyklus oder des Aminosäure-Exports zu überexprimieren.

So kann beispielsweise für die Herstellung von L-Lysin
- gleichzeitig das für die Dihydrodipicolinat-Synthase kodierende dapA-Gen (EP-B 0 197 335), oder
- gleichzeitig das für die Glyceraldehyd-3-Phosphat Dehydrogenase kodierende gap-Gen (Eikmanns (1992). Journal of Bacteriology 174:6076-6086), oder
- gleichzeitig das für die Triosephosphat Isomerase kodierende tpi-Gen(Eikmanns (1992). Journal of Bacteriology 174:6076-6086), oder
- gleichzeitig das für die 3-Phosphoglycerat Kinase kodierende pgk-Gen(Eikmanns (1992). Journal of Bacteriology 174:6076-6086), oder
- gleichzeitig das für die Pyruvat Carboxylase kodierende pyc-Gen(Eikmanns (1992). Journal of Bacteriology 174:6076-6086), oder
- gleichzeitig das für den Lysin-Export kodierende lysE-Gen (DE-A-195 48 222)
- gleichzeitig das für die Malat-Chinon-Oxidoreduktase kodierende mqo-Gen (Molenaar et al. (1998), European Journal of Biochemistry 254: 395-403),
- das für das zwal-Gen (DE: 19959328.0, DSM 13115)
überexprimiert werden.

Weiterhin kann es für die Produktion von Aminosäuren, insbesondere L-Lysin, vorteilhaft sein, zusätzlich zur Verstärkung des gpm-Gens gleichzeitig
- das für die Phosphoenolpyruvat-Carboxykinase kodierende pck-Gen (DE 199 50 409.1, DSM 13047), oder
- das für die Glucose-6-Phosphat Isomerase kodierende pgiGen (US 09/396,478, DSM 12969), oder
- das für die Pyruvat-Oxidase kodierende poxB Gen (DE: 19951975.7, DSM 13114), oder
- das zwa2-Gen (DE: 19959327.2, DSM 13113)
abzuschwächen.

Weiterhin kann es für die Produktion von Aminosäuren, insbesondere L-Lysin, vorteilhaft sein, neben der Überexpression des gpm-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren, insbesondere L-Lysin, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose,-Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten werden Sauerstoff oder Sauerstoff haltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an Lysin gebildet hat. Dieses Ziel wird normalerweise innerhalb von 10 Stunden bis 160 Stunden erreicht.

Die Analyse von L-Lysin kann durch Anionenaustauschchromatographie mit anschließender Ninhydrin Derivatisierung erfolgen, so wie bei Spackman et al. (Analytical Chemistry, 30, (1958), 1190) beschrieben.

Folgende Mikroorganismen wurden bei der Deutschen Sammlung für Mikrorganismen und Zellkulturen (DSMZ, Braunschweig, Deutschland) am 17.04.2000 gemäß Budapester Vertrag hinterlegt:
- Corynebacterium glutamicum Stamm DSM5715/pXKgpmexp als DSM 13456
- Corynebacterium glutamicum Stamm DSM5715/pEC-XK als DSM 13455

Das erfindungsgemäße Verfahren dient zur fermentativen Herstellung von Aminosäuren, insbesondere L-Lysin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCosl (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 mg/l Ampicillin ausplattiert wurden. Nach Inkubation über Nacht bei 37°C wurden rekombinante Einzelklone selektioniert.

### Beispiel 2

### Isolierung und Sequenzierung des gpm-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5aMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 mg/l Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZerol-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID No. 1 dargestellt. Die Analyse der Nukleotidsequenz ergab ein offenes Leseraster von 744 Basenpaaren, welches als gpm-Gen bezeichnet wurde. Das gpm-Gen kodiert für ein Protein von 248 Aminosäuren.

### Beispiel 3

### Herstellung eines Shuttle- Expressionsvektors pXKgmpexp zur Verstärkung des gpm-Gens in C. glutamicum

### 3.1. Klonierung des gpm Gens

Aus dem Stamm ATCC 13032 nach der Methode von Eikmanns et al. (Microbiology 140: 1817-1828 (1994)) chromosomale DNA isoliert. Aufgrund der aus Beispiel 2 für C. glutamicum bekannten Sequenz des gpm-Gens wurden die folgenden Oligonukleotide für die Polymerase Kettenreaktion ausgewählt:

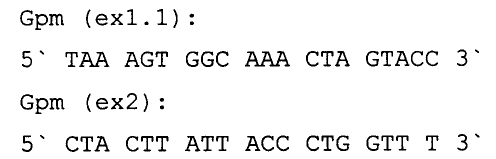

Die dargestellten Primer wurden von der Firma ARK Scientific GmbH Biosystems (Darmstadt, Deutschland) synthetisiert und nach der Standard-PCR-Methode von Innis et al. (PCR protocols. A guide to methods and applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion ermöglichen die Primer die Amplifikation eines ca. 0,77 kb großen DNA-Fragmentes, welches das gpm-Gen trägt.

Das ca. 0,77 kb große gpm Fragment wurde aus dem Agarosegel mit dem QiaExII Gel Extraction Kit (Product No. 20021, Qiagen, Hilden, Germany) isoliert.

### 3.2. Konstruktion des Shuttle - Vektors pEC-XK99E

Nach dem Stand der Technik wurde der E. coli - C. glutamicum Shuttle - Vektor pEC-XK99E konstruiert. Der Vektor enthält die Replikationsregion rep des Plasmides pGA1 einschließlich des Replikationseffectors per (US-A-5,175,108; Nesvera et al., Journal of Bacteriology 179, 1525-1532 (1997)), das Kanamycin-Resistenzgen aph(3')-IIa aus Escherichia coli (Beck et al. (1982), Gene 19: 327-336), den Replikationsursprung, den trc-Promotor, die Terminationsregionen T1 und T2, das lacI^{q}-Gen (Repressor des lac-Operons von E.coli) und eine Mehrfachklonierschnittstelle (multiple cloning site, mcs) (Norrander, J.M. et al. Gene 26, 101-106 (1983)) des Plasmids pTRC99A (Amann et al. (1988), Gene 69: 301-315).

Der konstruierte E. coli - C. glutamicum Shuttle - Vektor pEC-XK99E wurde mittels Elektroporation (Liebl et al., 1989, FEMS Microbiology Letters, 53:299-303) in C. glutamicum DSM5715 transferiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 25 mg/l Kanamycin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit der Restriktionsendonuklease HindIII geschnitten und das Plasmid durch anschließende Agarosegel-Elektrophorese überprüft.

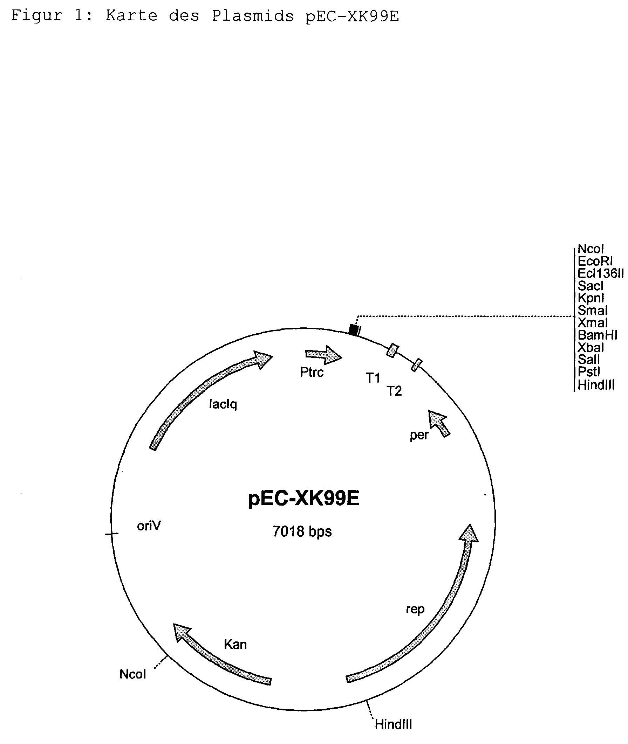

Das so erhaltene Plasmidkonstrukt wurde als pEC-XK99E (Figur 1) bezeichnet. Der durch Elektroporation des Plasmides pEC-XK99E in den C.glutamicum-Stamm DSM5715 erhaltene Stamm wurde DSM5715/pEC-XK99E genannt und als DSM 13455 bei der Deutschen Sammlung für Mikroorganismen und Zellkulturen (DSMZ, Braunschweig, Deutschland) gemäß Budapester Vertrag hinterlegt.

### 3.3. Klonierung von gpm im E. coli-C. glutamicum Shuttle Vektor pEC-XK99E

Als Vektor wurde der in Beispiel 3.2 beschriebene E. coli-C. glutamicum Shuttle-Vektor pEC-XK99E verwendet. DNA dieses Plasmids wurde mit dem Restriktionsenzymen Ecl136II vollständig gespalten und anschließend mit shrimp alkalischer Phosphatase (Roche Diagnostics GmbH, Mannheim, Deutschland, Produktbeschreibung SAP, Product No. 1758250) dephosphoryliert.

Das in Beispiel 3.1 beschriebene, mittels PCR gewonnene 0,77 kb große gpm-Fragment wurde mit dem vorbereiteten Vektor pEC-XK99E gemischt und der Ansatz mit T4-DNA-Ligase (Amersham Pharmacia, Freiburg, Deutschland, Produktbeschreibung T4-DNA-Ligase, Code no.27-0870-04) behandelt. Der Ligationsansatz wurde in den E. coli Stamm DH5a (Hanahan, In: DNA cloning. A practical approach. Vol. I. IRL-Press, Oxford, Washington DC, USA) transformiert. Die Selektion von Plasmid-tragenden Zellen erfolgte durch Ausplattieren des Transformationsansatzes auf LB-Agar (Lennox, 1955, Virology, 1:190) mit 25 mg/l Kanamycin. Nach Inkubation über Nacht bei 37°C wurden rekombinante Einzelklone selektioniert. Plasmid DNA wurde aus einer Transformante mit dem Qiaprep Spin Miniprep Kit (Product No. 27106, Qiagen, Hilden, Germany) nach Herstellerangaben isoliert und mit den Restriktionsenzymen EcoRI und XbaI gespalten, um das Plasmid durch anschließende Agarosegel-Elektrophorese zu überprüfen. Das erhaltene Plasmid wurde pXKgpmexp genannt. Es ist in Figur 2 dargestellt.

### Beispiel 4:

### Transformation des Stammes DSM5715 mit dem Plasmid pXKgpmexp

Der Stamm DSM5715 wurde mit dem Plasmid pXKgpmexp unter Anwendung der von Liebl et al., (FEMS Microbiology Letters, 53:299-303 (1989)) beschriebenen Elektroporationsmethode transformiert Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 25 mg/l Kanamycin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit den Restriktionsendonukleasen EcoRI und XbaI geschnitten und das Plasmid durch anschließende Agarosegel-Elektrophorese überprüft. Der erhaltene Stamm wurde DSM5715/pXKgpmexp genannt.

Folgender Mikroorganismus wurde bei der Deutschen Sammlung für Mikrorganismen und Zellkulturen (DSMZ, Braunschweig, Deutschland) gemäß Budapester Vertrag hinterlegt:
- Corynebacterium glutamicum Stamm DSM5715/pXKgpmexp als DSM 13456.

### Beispiel 5:

### Herstellung von Lysin

Der in Beispiel 4 erhaltene C. glutamicum Stamm DSM5715/pXKgpmexp wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz-Agar mit Kanamycin (25 mg/l)) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wurde auf pH 7.4
eingestellt

Diesem wurde Kanamycin (25 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,1 betrug. Für die Hauptkultur wurde das Medium MM verwendet.

| Medium MM | |
|---|---|
| CSL (Corn Steep Liquor) | 5 g/l |
| MOPS (Morpholinopropansulfonsäure) | 20 g/l |
| Glucose (getrennt autoklaviert) | 50 g/l |
| | |
| (NH₄)₂SO₄ | 25 g/l |
| KH₂PO₄ | 0, 1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| L-Leucin. (sterilfiltriert) | 0,1 g/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 72 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| DSM5715 | 6,8 | 13,68 |
| DSM5715/pXKgpmexp | 7,3 | 14,35 |

Folgende Figuren sind beigefügt:
Figur 1: Karte des Plasmids pEC-XK99E
Figur 2: Karte des Plasmids pXKgpmexp

Die verwendeten Abkürzungen und Bezeichnungen haben folgende Bedeutung:
- per:: Gen zur Kontrolle der Kopienzahl aus pGA1
- oriE:: Plasmidkodierter Replikationsursprung von E. coli
- rep:: Plasmidkodierter Replikationsursprung aus C. glutamicum Plasmid pGAl
- Ptrc:: trc-Promotor aus pTRC99A
- T1, T2:: Terminatorregionen 1 und 2 aus pTRC99A
- lacIq:: Repressor-Gen des Lac-Operon
- Kan:: Resistenzgen für Kanamycin
- gpm:: gpm-Gen von C.glutamicum

- EcoRI:: Schnittstelle des Restriktionsenzyms EcoRI
- Ecl136II:: Schnittstelle des Restriktionsenzyms Ecl136II
- HindIII:: Schnittstelle des Restriktionsenzyms HindIII
- KpnI:: Schnittstelle des Restriktionsenzyms KpnI
- SalI:: Schnittstelle des Restriktionsenzyms SalI
- SmaI:: Schnittstelle des Restriktionsenzyms SmaI
- PstI:: Schnittstelle des Restriktionsenzyms PstI
- BamHI:: Schnittstelle des Restriktionsenzyms BamHI
- NcoI:: Schnittstelle des Restriktionsenzyms NcoI
- XbaI:: Schnittstelle des Restriktionsenzyms XbaI
- XmaI:: Schnittstelle des Restriktionsenzyms XmaI
- SacI:: Schnittstelle des Restriktionsenzyms SacI

## Patentansprüche

1. Isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
e) Polynukleotid, enthaltend mindestens 15 aufeinanderfolgende Basen der Polynukleotidsequenz von a), b) oder c).

2. Polynukleotid gemäß Anspruch 1,
wobei das Polynukleotid eine in coryneformen Bakterien replizierbare, bevorzugt rekombinante DNA ist.

3. Polynukleotid gemäß Anspruch 1,
wobei das Polynukleotid eine RNA ist.

4. Polynukleotid gemäß Anspruch 2,
enthaltend die Nukleinsäuresequenz wie in SEQ ID No. 1 dargestellt.

5. Replizierbare DNA gemäß Anspruch 2,
enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

6. Polynukleotidsequenz gemäß Anspruch 2, das für ein Polypeptid kodiert, das die Aminosäuresequenz in SEQ ID No. 2 darstellt, enthält.

7. Verfahren zur fermentativen Herstellung von L-Aminosäuren, insbesondere L-Lysin,
**dadurch gekennzeichnet,**
daß man folgende Schritte durchführt:
a) Fermentation der die L-Lysin produzierenden coryneformen Bakterien, in denen man zumindest das gpm-Gen oder dafür kodierende Nukleotidsequenzen verstärkt, insbesondere überexprimiert.
b) Anreicherung von der L-Aminosäure im Medium oder in den Zellen der Bakterien und
c) Isolieren von der L-Aminosäure.

8. Verfahren gemäß Anspruch 7,
**dadurch gekennzeichnet,**
daß man Bakterien einsetzt, in denen man zusätzlich weitere Gene des Biosyntheseweges der gewünschten L-Aminosäure verstärkt.

9. Verfahren gemäß Anspruch 7,
**dadurch gekennzeichnet,**
daß man Bakterien einsetzt, in denen die Stoffwechselwege zumindest teilweise ausgeschaltet sind, die die Bildung des L-Lysins verringern.

10. Verfahren gemäß Anspruch 7,
**dadurch gekennzeichnet,**
daß man einen mit einem Plasmidvektor transformierten Stamm einsetzt, und der Plasmidvektor die für das gpm-Gen kodierende Nukleotidsequenz trägt.

11. Verfahren gemäß einem oder mehreren der Ansprüche 7 bis 10,
**dadurch gekennzeichnet,**
daß man coryneforme Bakterien verwendet, die L-Lysin herstellen.

12. Verfahren gemäß Anspruch 8,
**dadurch gekennzeichnet,**
daß gleichzeitig das für die Dihydrodipicolinat-Synthase kodierende dapA-Gen überexprimiert wird.

13. Verfahren gemäß Anspruch 8,
**dadurch gekennzeichnet,**
daß gleichzeitig ein S-(2-Aminoethyl)-Cystein-Resistenz vermittelndes DNA-Fragment amplifiziert wird.

14. Verfahren gemäß Anspruch 8,
**dadurch gekennzeichnet,**
daß gleichzeitig das für die Gyceraldehyd-3-Phosphat Dehydrogenase kodierende gap-Gen überexprimiert wird.

15. Verfahren gemäß Anspruch 8,
**dadurch gekennzeichnet,**
daß gleichzeitig das für die Triosephosphat Isomerase kodierende tpi-Gen überexprimiert wird.

16. Verfahren gemäß Anspruch 8,
**dadurch gekennzeichnet,**
daß gleichzeitig das für die 3-Phosphatglycerat Kinase kodierende pgk-Gen überexprimiert wird.

17. Verfahren gemäß Anspruch 8,
**dadurch gekennzeichnet,**
daß gleichzeitig das für die Pyruvat Carboxylase kodierende pyc-Gen überexprimiert wird.

18. Verwendung von Polynukleotidsequenzen gemäß Anspruch 1 als Primer zur Herstellung der DNA von Genen, die eine dem opcA-Gen entsprechende Wirkung entfalten, durch die Polymerase-Kettenreaktion.

19. Verwendung von Polynukleotidsequenzen gemäß Anspruch 1 als Hybridisierungssonde.

20. Coryneforme Mikroorganismen, insbesondere der Gattung Corynebacterium, transformiert durch die Einführung der replizierbaren DNA gemäß einem der Ansprüche 1 oder 5.
